# EUROPEAN PATENT SPECIFICATION

(11) **EP 2 398 728 B1**
(45) Date of publication and mention of the grant of the patent: **03.04.2013**
(21) Application number: 10710428.3
(22) Date of filing: 16.02.2010
(51) Int. Cl.: B65H 63/032, G01N 21/89, G01N 33/36

(54) **PROGRAMMABLE SENSOR SUITABLE FOR CONTROLLING YARN FEED TO A TEXTILE MACHINE AND METHOD FOR THE PROGRAMMING THEREOF**
PROGRAMMIERBARER SENSOR ZUR STEUERUNG EINER GARNZUFUHR AN EINE TEXTILMASCHINE UND VERFAHREN ZU DESSEN PROGRAMMIERUNG
CAPTEUR PROGRAMMABLE ADAPTÉ POUR CONTRÔLER LA FOURNITURE D'UN FIL À UNE MACHINE TEXTILE ET PROCÉDÉ POUR LA PROGRAMMATION DE CE CAPTEUR

(30) Priority: 19.02.2009 IT MI20090219
(43) Date of publication of application: 28.12.2011
(73) Proprietor: BTSR INTERNATIONAL S.P.A., 21057 Olgiate Olona (VA) (IT)
(72) Inventor: BAREA, Tiziano, I-21052 Busto Arsizio (VA) (IT)
(74) Representative: Ripamonti, Enrico
(86) International application number: PCT/IB2010/000322
(87) International publication number: WO 2010/095023

(56) References cited:
- EP-A1- 0 619 261
- EP-A2- 0 950 742
- WO-A1-2005/078384
- US-A- 5 015 867
- US-B1- 6 476 619

## Description

The subject of the present invention is a sensor for controlling yarn feed to a textile machine according to the preamble of the main claim. A further subject of the invention is a method for choosing a particular and selected operating mode of said sensor.

Various types of sensor are known suitable for detecting at least one specific characteristic of a thread or yarn fed to a textile machine: for example, sensors are known suitable for detecting the running or state of rest of the yarn and/or for detecting the speed and/or tension of said yarn during its movement towards the textile machine. Said sensors are provided with a casing positioned on a supporting structure of the machine or in the vicinity of the latter; the casing is furthermore provided with known means (mechanical, optical and/or electronic) suitable for detecting the above-mentioned characteristic of the yarn. Display means and buttons are also provided in the casing suitable for enabling a user to set or re-set data relative to the tension and/or speed of the yarn to be controlled in addition to other operating parameters of the device considered acceptable for correct feed of the yarn to a textile machine.

A known solution of this type is contained in EP619261 which describes a method and a device for adjusting and maintaining a predefined tension of a yarn fed to a textile machine.

Said patent text does not describe, however, the possibility of the operator setting a plurality of operating modes of the yarn control device, intervening on the internal operating parameters of the device itself according to specific varying operating conditions (such as the type of yarn or its section).

Usually a textile machine comprises many sensors of the type cited, all connected to a device for controlling correct feed of the yarn to the machine. The data detected by each sensor are transferred, via known methods, to means of said device for control of the data detected which, according to the latter, intervene on the sensor operating modes (for example by causing the sensor to modify the yarn running tension) or on the textile machine (for example by stopping operation thereof if the sensor detects an incorrect state of movement of the yarn, or an absence of the latter).

Said known solutions therefore concern sensors of a characteristic of the yarn which are substantially detectors of said characteristic and can operate effectively only if connected to a yarn feed control device, which may or may not be part of the usual operating control device of the textile machine. A user of the latter who wishes to obtain a product of acceptable quality must therefore adopt a device of the type cited (which substantially comprises one or more sensors connected to a yarn feed control unit separate from the sensor) or must possess a textile machine with "electronics" (i.e. the set of all the electrical/electronic components that permit correct operation) already provided with interface for the above-mentioned sensors.

US6476619 describes a method and a device for accurately measuring and calculating at high speed the humidity and density contents of fibrous material, homogeneous or non-homogeneous, via the use of a microwave resonator device and calculation algorithms. With this device, automatic feedback is obtained on the density quality of the fibrous material, via measurement, control and regulation of the humidity content, temperature and feed speed. Said quality control feedback and regulation are automatically actuated via a connection between a central processing unit provided in the device and a machine separating and processing the CARD fibres (CARD fiber separating machine).

The algorithms described are calculation algorithms (and not algorithms for control of the yarn process) suitable for permitting processing of the data received by the central unit.

Said patent does not comprise the possibility of a user or an operator selecting a plurality of intervention modes (according to predefined control algorithms) on the fibrous material.

From the above it appears evident that the solutions in the state of the art are costly (if, in addition to the sensors, the user also has to acquire the above-mentioned control device or even an entire textile machine with said device fitted inside) and also penalise the user if he cannot modify the electronics of the textile machine he is already operating in order to equip it with the above-mentioned control device.

The object of the present invention is to provide a sensor for controlling the feed of a thread or a yarn to a textile machine which can be selectively used according to different operating modes or according to different control algorithms, without the need to connect the same sensor to a separate operating control and command unit.

A further object is to provide a sensor of the type cited which permits control of the feed of the thread or yarn to a textile machine even if the control electronics of the latter are not provided with a device for controlling the feed of the thread or yarn to the machine, it being possible to use said sensor completely independently and without the need for connection to a different and separate independent yarn feed control device.

In other words, the object of the invention is to provide a sensor suitable for controlling yarn feed with at least one of its characteristics or parameters (tension or speed) maintained constant which allows for modification of the control algorithm of said feed according to different needs such as type or section of the yarn.

A further object is to provide a sensor of the type cited which can be produced according to the procedures and conformations of the sensors currently produced, thus avoiding a significant increase in the production costs of the sensor.

A further object is to provide a sensor of the type cited which is simple to use.

A further object of the invention is to provide a method for simply and rapidly programming a thread or yarn feed control sensor of the type cited above according to predefined and different operating parameters such as to permit a plurality of different yarn control modes.

These and further objects which will be evident to a person skilled in the art are obtained by a sensor and a method as claimed in the attached claims.

For a better understanding of the present invention, the following drawings are attached, purely by way of non-limiting example, in which:
figure 1 shows a generic diagram of a sensor for controlling yarn feed to a textile machine obtained according to the present invention;
figure 2 shows a flow diagram illustrating a method according to the invention; and
figure 3 shows in a perspective view an example of embodiment and use of a sensor according to the invention.

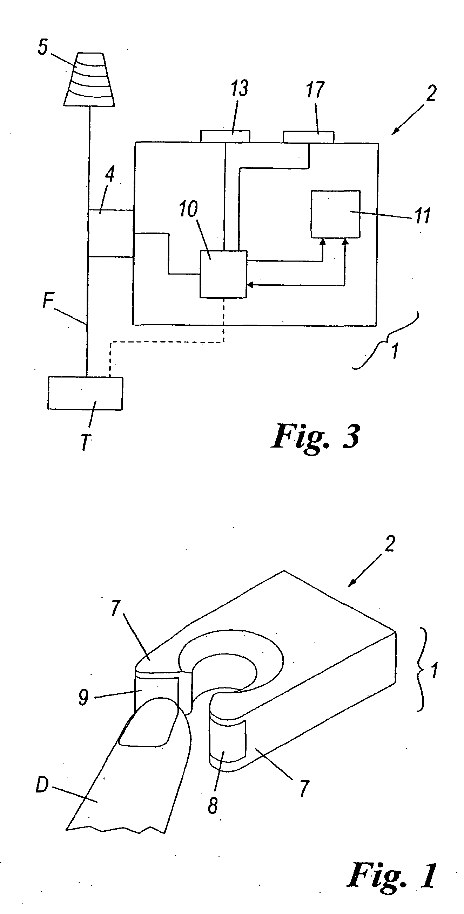

With reference to the figures cited, a sensor suitable for controlling the correct feed of a yarn F to a textile machine T is indicated generically by 1 and comprises a casing 2 suitable for being combined with an adequate support (not shown) positioned in the vicinity of the textile machine; said support may or may not be part of the latter. The sensor 1 has usual detection means 4 cooperating with the yarn F after, for example, it has been unwound from a corresponding spool 5 and during its movement or running towards the machine T. Said detection means can be of the mechanical type, such as one or more movable arms overhanging the casing 2 or a rotating element positioned on one face of the latter; optical as shown in figure 3 where the sensor 1 (of known type and sold by the applicant under the name IS3) comprises a casing 2 with opposite arms 7 provided at the ends with optical elements 8 and 9 suitable for detecting at least the presence of the yarn between them; electrical, electronic or defined by components that exploit mechanical and electrical characteristics (such as piezoelectric detectors). The above-mentioned detection means, being of known type and easily accessible in the state of the art, will not be further described.

According to the invention, said sensor 1 comprises a means for control of said detection and means suitable for modifying the modes of said control according to a plurality of control algorithms pre-set and stored in the sensor; the sensor 1 also comprises selector means suitable for allowing the selection of one of said algorithms so that the sensor can permit a plurality of different yarn control modes according to different needs such as, for example, different yarn section, different yarn materials and yarns with different elasticity and elongation characteristics.

Thanks to the invention, using the same sensor 1, an operator can select different algorithms or control methods, also operating with yarns having different characteristics.

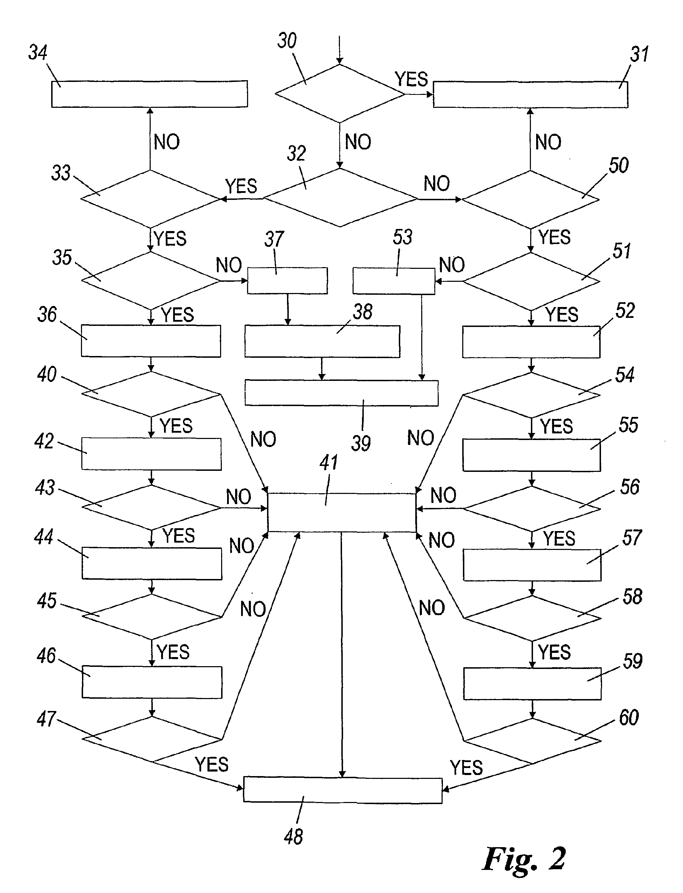

More specifically, inside the casing 2 of the sensor 1 there is a control unit 10, preferably and advantageously microprocessor-operated, defining the control means of the characteristic identified by the detection means in addition to the means suitable for modifying said control mode. The unit 10 is connected to the detection means and to a memory unit 11 containing the different control algorithms (based on predefined operating parameters differing from algorithm to algorithm) according to which different possible yarn feed controls are performed. The unit 10 dialogues with said memory unit (or simply "memory") 11 according to the intervention of a user on the selector means defined by a selector 13 which can be specifically provided on the casing 2 for the selection of one of various operating algorithms or which can be a functional component of the sensor 1 used so as to select the above-mentioned algorithm obtained mechanically (for example via a movable button), electrically (for example via a touch button) or optically (for example via the interruption of an "optical contact" between the optical elements 8 and 9 of the sensor of figure 2). Said intervention is defined by the method according to the invention which will be described in relation to figures 2 and 3.

It is firstly assumed that the memory 11 contains four different operating algorithms of the sensor 1 (for example a sensor suitable for detecting the yarn feed status), said algorithms being chosen, for example, in order to allow the sensor to operate effectively with yarns of different section or different material or different elasticity and elongation, or with the same yarn but in different operating conditions (according to variations in the yarn pick-up speed or draw). In order to select the desired operating algorithm (for example chosen according to the yarn being worked), the user intervenes on the selector 13: for example, in the case of the sensor of figure 2, the user places his finger D between the optical elements 8 and 9 thus allowing the unit 10 to detect the presence thereof. This action corresponds to pressing a button or a touch key. It should be noted that by maintaining the action on the selector 13 for a brief period (for example 1 or 2 seconds and in any case less than a pre-established value t, for example 3 seconds), the unit 10 switches the status of the sensor 1; said switching is visually identified by the activation of an illuminated display device 17 (for example a LED) and/or an acoustic element, connected to the unit 10. In this way, the status of the sensor passes for example from on (LED activated) to off (LED de-activated) or vice versa. If the sensor 1 is already on, a series of warnings is generated before switching off, for example blinkings of the LED, equal to the number of the operating algorithm currently followed by the sensor 1 during its operation.

The method according to the invention begins (block 30 of figure 3) with evaluation of the status of the yarn F, i.e. whether the yarn is running or not. If the yarn is already moving, "programming" of the sensor 1 (i.e. the selection of a particular and predefined operating algorithm present in the memory 11) is stopped (block 31). If, on the other hand, the yarn is at a standstill, the program goes to block 32 where a check is performed to verify whether the sensor 1 is activated (on) or de-activated (off). According to said check, one of two different sequences can be followed, shown in figure 3.

If the sensor 1 is on, a check is performed to verify action on the selector 13 (block 33): if it is not detected, the programming is stopped (block 34).

If, on the other hand, an action is performed on the selector 13, a check is carried out to verify (block 35) whether said action occurs for a period longer than the pre-established value t cited above. If so, the program goes on to block 36 where the first of the four algorithms stored is selected (and the display device 17 is activated one single time - corresponding to the algorithm number 1); if the check is negative, the program certifies that the action on the selector 13 represents the desire to de-activate the sensor 1 and goes on to block 37 (sensor switch-off) and then to block 38 where the series of light (and/or acoustic) signals is generated corresponding in number to the number of the algorithm currently followed by the sensor for control of the yarn. The program then goes on to block 39 where it terminates.

If on the other hand the program went to block 36, a check is subsequently performed (block 40) to verify whether the action on the selector 13 is continuing: if not, the program goes on to block 41 where the selected operating algorithm is stored. If the answer is positive, the program goes on to block 42 corresponding to the selection of program number 2 with consequent dual activation of the display device 17.

The program then checks (block 43) whether the action on the selector 13 persists: if not, it goes on to block 41; if so, it certifies selection of the third operating algorithm (block 44) with consequent triple activation of the display device 17.

The program then checks again (block 45) whether the action on the selector 13 persists: if not, it goes on to block 41; if so, it certifies selection of the fourth operating algorithm (block 46) with quadruple activation (in time sequence) of the display device 17.

The program then checks (block 47) whether the action on the selector 13 persists: if the check is negative, it goes on to block 41 (storage of the selected operating algorithm); if positive, it exits from the "programming" or selection of the algorithm (block 48).

An analogous procedure is followed if the check performed in block 32 indicates that the sensor is off. This involves transition to block 50 corresponding to block 33 cited above. If there is no action on the selector 13, the program returns to block 31, otherwise it goes to block 51 corresponding to block 35. If the action on the selector lasts beyond the pre-set time t (cited above), the program goes on to block 52 corresponding to block 36, otherwise it goes to block 53, in which the sensor 1 is activated, and from there to block 39.

If the program is in the phase or step of the method corresponding to block 52, it follows the procedure indicated by the blocks 54, 55, 56, 57, 58, 59, 60 corresponding to the procedure comprising the blocks described above 40, 42, 43, 44, 45, 46 and 47 which should be referred to for the sake of brevity.

The above description highlights that, both with the sensor activated and de-activated, the action on the selector 13 for a period longer than a predefined time t (for example equal to 3 seconds) triggers in sequence the various implementation phases of the operating algorithm selection method. Each phase (i.e. the corresponding algorithm) is highlighted by activation of the display device 17 for a period corresponding to the algorithm selected (in the example, one, two, three or four times). It is sufficient to terminate the action on the selector 13 to select the operating algorithm of the sensor 1 chosen at that particular moment (block 41).

It should be noted that even with the sensor de-activated, after selection of its operating mode ("programming"), it is automatically activated after the yarn F has started to run, cooperating with the yarn for a predefined period, for example equal to the period or time t cited above. Signalling of activation of the sensor is indicated by activation of the display device 17.

The invention therefore allows the use of one single yarn feed control sensor which is able to operate according to different operating algorithms, pre-set and stored in an internal memory unit and selected according to different needs, for example yarn type or section; there is no need for any further sensor control devices, which are certainly more costly and not always easy to position on or near the textile machine if they are independent of it, or for the machine control electronics to be pre-set for said sensor control. This allows the yarn to be controlled according to different operating modes without having to intervene on the machine or without having to equip the latter with sensors and correlated control devices which can also be fairly costly; said yarn control is obtained alternatively with one single sensor which incorporates the functions of a component suitable for controlling parameters of the yarn fed and the possibility of varying, in a predefined manner, the control modes of said parameters. This involves a simplification of both the machine and the yarn control "system", i.e. the set of all the components and devices necessary for correct feed of the thread or yarn to the above-mentioned machine.

It should be noted that the sensor can also be pre-set to intervene directly on the machine T to block the operation thereof if the sensor detects an anomaly in the characteristic of the thread or yarn controlled during feeding to said machine. This is indicated in figure 1.

A particular embodiment of the invention has been described. Others are possible in the light of the preceding description and comprise, for example, sensors operating on different characteristics of the yarn or sensors which can operate according to algorithms (in a number higher than four); said embodiments must be considered as falling within the scope of the following claims.

## Claims

1. Sensor (1) for controlling thread or yarn (F) feed to a textile machine (T) comprising a body or casing (2) suitable for being arranged on a support of the machine (T) or positioned near to it and having detection means (4) suitable for detecting at least one feed characteristic of the yarn (F) to said machine, **characterised in that** in the casing or body (2) of the sensor, means (10) are provided for control of said detection and suitable for modifying the modes of said control according to a plurality of control algorithms stored in memory means (11) associated with said casing (2) associated with the latter, selector means (13) being also provided suitable for allowing a user to choose one of said algorithms, thus selecting the desired operating mode of the sensor during its yarn feed control, said sensor thus permitting control of the yarn feed according to a plurality of modes corresponding to the different control algorithms referred to above and stored in the sensor.

2. Sensor as claimed in claim 1, **characterised in that** said control means suitable for modifying the modes of said control are a microprocessor unit (10), said unit being connected to a memory unit (11) defining the memory means and to the detection means (4).

3. Sensor as claimed in claim 1, **characterised in that** it comprises a display device (17) for displaying the selection of a particular algorithm from among the operating algorithms stored.

4. Sensor as claimed in claim 1, **characterised in that** the selector means (13) are elements specifically provided on the casing (2) of the sensor, said element being mechanical or optical or electrical/electronic such as a touch key

5. Sensor as claimed in claim 1, **characterised in that** the selector means (13) are at least one element (7, 8) of the sensor suitable for permitting the usual function for control of the yarn feed to the textile machine (T).

6. Sensor as claimed in claim 5, **characterised in that** the selector means (13) are optical elements (7, 8) suitable for permitting the determination of a dimensional characteristic of the yarn (F) fed to the textile machine (T).

7. Method for selecting an operating mode of a sensor (1), suitable for controlling the feed of thread or yarn (F) to a textile machine (T), from a plurality of different operating modes that can be implemented with said sensor, each of said modes being obtained with the sensor (1) operating according to a corresponding control algorithm chosen from a plurality of different algorithms corresponding to the various operating modes and based on pre-defined controlled parameters of the yarn (1) specific for each mode, said method being **characterised in that** it comprises:
- at least one intervention by a user on an operating mode selector (13) associated with a casing of the sensor (1), said intervention being performed with different continuous periods of time lasting beyond a pre-determined period (t), different durations of said periods defining a selection of different control algorithms stored in memory means (11) (44) positioned inside a casing (2) of the sensor (1) or different operating modes of the sensor (1),
- indication of the choice of a particular operating algorithm from the plurality of algorithms selectable,
- waiting for a pre-defined period and
- implementation of the operating mode corresponding to the algorithm selected by the sensor (1).

8. Method as claimed in claim 7, **characterised in that** the cooperating with a control unit (10) for controlling memory means (11) are operation of the sensor (1) which is also inside said casing and connected to the above-mentioned selector (13); said control unit (10) is connected to an element (4) for detecting a controlled characteristic of the yarn and cooperating with it, the aforesaid control unit (10) activating a display device (17) suitable for providing an indication of the selected operating algorithm desired by the user and, after waiting for the pre-defined period, permitting operation of the sensor (1) according to the particular algorithm selected by intervention on the selector (13).

9. Method as claimed in claim 8, **characterised by** continued intervention on the selector (13), the following being activated in succession in said period of continuous intervention: the display device (17) thus indicating in sequence the various possible operating algorithms for the sensor, the control unit (10) allowing operation of the sensor according to an algorithm only when the intervention on the selector (13) terminates and a period of time has elapsed longer than a predetermined period predefined after said termination of the action on the selector (13), said operation occurring according to specific operating parameters read in the memory means (11) and corresponding to the operating algorithm indicated by the last activation of the above-mentioned display device.

10. Method as claimed in claim 9, **characterised in that** selection of the operating algorithm of the sensor (1) occurs both when the latter is activated and when it is de-activated, said sensor being automatically activated if previously not functioning after a movement of said thread or yarn (F) towards the textile machine (T) lasting longer than a pre-set period of time, said automatic activation being indicated by the display device (17).

11. Method as claimed in claim 7, **characterised in that** the textile machine (T) will be stopped by the sensor (1) if it detects a difference between the datum detected of the parameter or characteristic of the thread or yarn controlled and the stored parameter of the operating algorithm according to which said sensor is operating.

## Patentansprüche

1. Ein Sensor (1) zur Kontrolle der Zufuhr eines Fadens oder Garns (F) zu einer Textilmaschine (T), die einen Körper oder ein Gehäuse (2) umfasst, das geeignet ist, an einer Halterung der Maschine (T) angeordnet beziehungsweise in ihrer Nähe positioniert zu werden und das Erkennungselemente (4) aufweist, welche geeignet sind, mindestens eine der Zufuhreigenschaften des Garns (F) zu der genannten Maschine festzustellen, **dadurch gekennzeichnet, dass** in dem Gehäuse oder Körper (2) des Sensors Elemente (10) zur Kontrolle dieser Erkennung vorgesehen und geeignet sind, die Art und Weisen dieser genannten Kontrolle gemäß einer Vielzahl von Kontrollalgorithmen zu ändern, welche in Speicherelementen (11) gespeichert sind, die mit dem genannten Gehäuse (2) verbunden sind, welches wiederum mit letzteren verbunden ist, wobei auch Auswahlelemente (13) vorgesehen sind, die geeignet sind, einem Anwender die Wahl eines der genannten Algorithmen zu gestatten, wodurch der gewünschte Betriebsmodus des Sensors während seiner Garnzufuhrkontrolle ermöglcht wird, und der genannte Sensor auf diese Weise die Kontrolle der Garnzufuhr gemäß einer Vielzahl von Art und Weisen gestattet, welche jeweils den einzelnen, oben erwähnten und im Sensor gespeicherten Kontrollalgorithmen entsprechen.

2. Ein Sensor gemäß Anspruch 1, **dadurch gekennzeichnet, dass** es sich bei den genannten Kontrollelemente, die geeignet sind, die Art und Weisen der genannten Kontrolle zu ändern, um eine Mikroprozessoreinheit (10) handelt, wobei die genannte Einheit mit einer Speichereinheit (11) verbunden ist, welche die Speicherelemente definiert, sowie mit den jeweiligen Erkennungselementen (4).

3. Ein Sensor gemäß Anspruch 1, **dadurch gekennzeichnet, dass** er eine Anzeigevorrichtung (17) zur Anzeige der Auswahl eines besonderen Algorithmus unter den gespeicherten Betriebsalgorithmen umfasst.

4. Ein Sensor gemäß Anspruch 1, **dadurch gekennzeichnet, dass** die Auswahlelemente (13) Elemente sind, die speziell am Gehäuse (2) des Sensors vorgesehen sind, wobei das genannte Element jeweils mechanischer, optischer oder elektrischer/elektronischer Natur wie eine Berührungstaste ist.

5. Ein Sensor gemäß Anspruch 1, **dadurch gekennzeichnet, dass** die Auswahlelemente (13) mindestens ein Element (7, 8) des Sensors sind, das geeignet ist, die gewöhnliche Funktion zur Kontrolle der Garnzufuhr zur Textilmaschine (T) zu gestatten.

6. Ein Sensor gemäß Anspruch 5, **dadurch gekennzeichnet, dass** die Auswahlelemente (13) optische Elemente (7, 8) sind, die geeignet sind, die Bestimmung einer Maßeigenschaft des Garns (F) zu gestatten, das der Textilmaschine (T) zugeführt wird.

7. Eine Methode zur Wahl eines Betriebsmodus eines Sensors (1), der geeignet ist, die Zufuhr eines Fadens oder Garns (F) zu einer Textilmaschine (T) zu kontrollieren, und zwar aus einer Vielzahl verschiedener Betriebsweisen, welche mit dem genannten Sensor durchgeführt werden können, wobei jede der genannten Weisen mit dem Sensor (1) erreicht wird, der nach einem entsprechenden Kontrollalgorithmus arbeitet, welcher aus einer Vielzahl unterschiedlicher Algorithmen ausgewählt wird, die jeweils den einzelnen Betriebsweisen entsprechen und auf vorbestimmten Kontrollparametern des Garns (1) beruhen, die für jeden Modus typisch sind, die genannte Methode **dadurch gekennzeichnet ist, dass** sie folgendes umfasst:
- Mindestens einen Eingriff durch einen Anwender an einem Betriebsmodus-Auswähler (13), der mit einem Gehäuse des Sensors (1) verbunden ist, wobei der genannte Eingriff jeweils mit verschiedenen kontinuierlichen Zeitperioden erfolgt, die über einen vorbestimmten Zeitraum (t) hinaus dauern, wobei die unterschiedlichen Längen der genannten Zeiträume eine Wahl unterschiedlicher Kontrollalgorithmen voraussetzt, welche in den Speicherelementen (11) gespeichert sind, die sich im Innern eines Gehäuses (2) des Sensors (1) befinden, beziehungsweise unterschiedlicher Betriebsweisen des Sensor (1);
- Die Angabe der Wahl eines besonderen Betriebsalgorithmus aus der Vielzahl wählbarer Algorithmen;
- Das Warten während eines vorbestimmten Zeitraums und
- Die Durchführung des Betriebsmodus, der dem durch den Sensor (1) gewählten Algorithmus entspricht.

8. Eine Methode gemäß Anspruch 7, **dadurch gekennzeichnet, dass** Speicherelemente (11) mit einer Kontrolleinheit (10) zur Kontrolle des Betriebs des Sensors (1) zusammenarbeiten, der sich ebenfalls im Innern des genannten Gehäuses befindet und mit dem oben erwähnten Auswähler (13) verbunden ist; die genannte Kontrolleinheit (10) ist mit einem Element (4) zur Erkennung einer kontrollierten Eigenschaft des Garns verbunden und arbeitet mit diesem zusammen, wobei die zuvor erwähnte Kontrolleinheit (10) eine Anzeigevorrichtung (17) einschaltet, die geeignet ist, nach der vorbestimmten Wartezeit eine Angabe des gewählten Betriebsalgorithmus zu liefern, der vom Anwender gewünscht wird, wobei der Betrieb des Sensors (1) nach dem besonderen Algorithmus gestattet wird, welcher durch die Betätigung des Wählers (13) gewählt wurde.

9. Eine Methode gemäß Anspruch 8, **gekennzeichnet durch** die wiederholte Betätigung des Wählers (13), wobei Folgendes in der Reihenfolge in dem genannten Zeitraum der ständigen Betätigung eingeschaltet wird: die Anzeigevorrichtung (17), wodurch der Reihe nach die einzelnen möglichen Betriebsalgorithmen für den Sensor angegeben werden, die Kontrolleinheit (10), welche den Betrieb des Sensors nach einem Algorithmus erst dann gestattet, wenn die Betätigung des Wählers (13) beendet wurde und ein Zeitraum verstrichen ist, der länger als eine vorbestimmte Zeit nach der genannten Beendung der Betätigung des Wählers (13) ist, wobei der genannte Betrieb jeweils nach spezifischen Betriebsparametern erfolgt, die in den Speicherelementen (11) gelesen werden und dem Betriebsalgorithmus entsprechen, der **durch** die letzte Einschaltung der oben erwähnten Anzeigevorrichtung angegeben wird.

10. Eine Methode gemäß Anspruch 9, **dadurch gekennzeichnet, dass** die Wahl des Betriebsalgorithmus des Sensors (1) sowohl erfolgt, wenn letzterer eingeschaltet ist, wie wenn er ausgeschaltet ist, wobei der genannte Sensor automatisch eingeschaltet wird, falls er zuvor nicht in Betrieb war, und zwar nach einer Bewegung des genannten Fadens oder Garns (F) in Richtung der Textilmaschine (T), die länger dauert als eine vorbestimmte Zeit, wobei diese automatische Einschaltung jeweils durch die Anzeigevorrichtung (17) angezeigt wird.

11. Eine Methode gemäß Anspruch 7, **dadurch gekennzeichnet, dass** die Textilmaschine (T) durch den Sensor (1) gestoppt wird, wenn er einen Unterschied zwischen der festgestellten Bezugsgröße des Parameters oder der Eigenschaft des kontrollierten Fadens oder des Garns und dem gespeicherten Parameter des Betriebsalgorithmus erkennt, nach dem der genannte Sensor arbeitet.

## Revendications

1. Capteur (1) pour commander l'acheminement de filé ou de fil (F) à une machine textile (T) comprenant un corps ou un boîtier (2) convenant pour être aménagé sur un support de la machine (T) ou positionné près de celle-ci et ayant des moyens de détection (4) convenant pour détecter au moins une caractéristique d'alimentation du fil (F) à ladite machine, **caractérisé en ce que**, dans le boîtier ou le corps (2) du capteur, il est prévu des moyens (10) pour commander ladite détection et qui conviennent pour modifier les modes de ladite commande conformément à une pluralité d'algorithmes de commande stockés dans des moyens de mémorisation (11) associés audit boîtier (2) associé à ces derniers, des moyens de sélection (13) étant également prévus, qui conviennent pour permettre à un utilisateur de choisir l'un desdits algorithmes, en choisissant donc le mode de fonctionnement souhaité du capteur au cours de sa commande d'alimentation en fil, ledit capteur permettant donc la commande de l'alimentation en fil selon une pluralité de modes correspondant aux différents algorithmes de commande mentionnés ci-dessus et stockés dans le capteur.

2. Capteur selon la revendication 1, **caractérisé en ce que** lesdits moyens de commande convenant pour modifier les modes de ladite commande sont une unité de microprocesseur (10), ladite unité étant connectée à une unité de mémorisation (11) définissant les moyens de mémorisation et aux moyens de détection (4).

3. Capteur selon la revendication 1, **caractérisé en ce qu'**il comprend un dispositif d'affichage (17) pour afficher la sélection d'un algorithme particulier parmi les algorithmes de fonctionnement stockés.

4. Capteur selon la revendication 1, **caractérisé en ce que** les moyens de sélection (13) sont des éléments spécifiquement prévus sur le boîtier (2) du capteur, ledit élément étant mécanique ou optique ou encore électrique/électronique, notamment une touche.

5. Capteur selon la revendication 1, **caractérisé en ce que** les moyens de sélection (13) sont au moins un élément (7, 8) du capteur qui convient pour permettre la fonction usuelle pour la commande de l'alimentation en fil à la machine textile (T).

6. Capteur selon la revendication 5, **caractérisé en ce que** les moyens de sélection (13) sont des éléments optiques (7, 8) convenant pour permettre la détermination d'une caractéristique dimensionnelle du fil (F) acheminé à la machine textile (T).

7. Procédé de sélection d'un mode de fonctionnement d'un capteur (1) convenant pour commander l'alimentation en filé ou en fil (F) à une machine textile (T) à partir d'une pluralité de différents modes de fonctionnement qui peuvent être mis en oeuvre avec ledit capteur, chacun desdits modes étant obtenu avec le capteur (1) qui fonctionne selon un algorithme de commande correspondant choisi parmi une pluralité de différents algorithmes correspondant aux divers modes de fonctionnement et basé sur des paramètres commandés prédéfinis du fil (1) spécifiques à chaque mode, ledit procédé étant **caractérisé en ce qu'**il comprend :
- au moins une intervention par un utilisateur sur un sélecteur de mode de fonctionnement (13) associé à un boîtier du capteur (1), ladite intervention étant effectuée avec différentes périodes de temps continues qui se déroulent au-delà d'une période prédéterminée (t), différentes durées desdites périodes définissant une sélection de différents algorithmes de commande ou stockées dans des moyens de mémorisation (11) positionnés à l'intérieur d'un boîtier (2) du capteur (1) ou différents modes de fonctionnement du capteur (1),
- l'indication du choix d'un algorithme de fonctionnement particulier parmi la pluralité d'algorithmes sélectionnables,
- l'attente d'une période prédéfinie, et
- la mise en oeuvre du mode de fonctionnement correspondant à l'algorithme choisi par le capteur (1).

8. Procédé selon la revendication 7, **caractérisé en ce que** les moyens de mémorisation (11) coopèrent avec une unité de commande (10) pour commander le fonctionnement du capteur (1) qui se trouve également à l'intérieur dudit boîtier et est raccordé au sélecteur précité (13) ; ladite unité de commande (10) est raccordée à un élément (4) pour détecter une caractéristique contrôlée du fil et coopérant avec celui-ci, l'unité de commande précitée (10) activant un dispositif d'affichage (17) convenant pour fournir une indication de l'algorithme de fonctionnement choisi souhaité par l'utilisateur et, après avoir attendu la période prédéfinie, permettre le fonctionnement du capteur (1) selon l'algorithme particulier choisi par l'intervention sur le sélecteur (13).

9. Procédé selon la revendication 8, **caractérisé par** une intervention continue sur le sélecteur (13), les opérations suivantes étant activées à la suite dans ladite période d'intervention continue : le dispositif d'affichage (17) indiquant donc en séquence les divers algorithmes de fonctionnement possibles pour le capteur, l'unité de commande (10) permettant le fonctionnement du capteur selon un algorithme uniquement lorsque l'intervention sur le sélecteur (13) se termine et qu'il s'est écoulé une période de temps plus longue qu'une période de temps prédéterminée prédéfinie après ladite fin de l'action sur le sélecteur (13), ledit fonctionnement se produisant selon des paramètres de fonctionnement spécifiques qui sont lus dans les moyens de mémorisation (11) et correspondent à l'algorithme de fonctionnement indiqué par la dernière activation du dispositif d'affichage mentionné ci-dessus.

10. Procédé selon la revendication 9, **caractérisé en ce que** la sélection de l'algorithme de fonctionnement du capteur (1) se produit à la fois lorsque ce dernier est activé et lorsqu'il est désactivé, ledit capteur étant automatiquement activé s'il ne fonctionne pas précédemment après un mouvement dudit filé ou dudit fil (F) vers la machine textile (T) qui dure plus longtemps qu'une période de temps pré-établie, ladite activation automatique étant indiquée par le dispositif d'affichage (17).

11. Procédé selon la revendication 7, **caractérisé en ce que** la machine textile (T) sera arrêtée par le capteur (1) s'il détecte une différence entre la date détectée du paramètre ou de la caractéristique du filé ou du fil commandé(e) et le paramètre stocké de l'algorithme de fonctionnement selon lequel ledit capteur fonctionne.
